# EUROPEAN PATENT APPLICATION

(11) **EP 3 984 988 A1**
(43) Date of publication of application: **20.04.2022**
(21) Application number: 20202486.5
(22) Date of filing: 19.10.2020
(51) Int. Cl.: C07C 51/12, C07C 67/08, C07C 53/08, C07C 69/14

(54) **RECOVERY PROCESS OF METHANOL WASTE**

(71) Applicant: Itelyum Regeneration S.p.A., 26854 Pieve Fissiraga (LO) (IT)
(72) Inventor: ABBATE, Paolo, 26854 Pieve Fissiraga Lodi (IT); GALLO, Francesco, 26854 Pieve Fissiraga Lodi (IT)
(74) Representative: Vatti, Francesco Paolo

(57) **Abstract**

Recovery process of methanol waste, which provides a first carbonylation step with CO starting from methanol; according to the invention, the product of said carbonylation is caused to react in a second step with methanol at a lower concentration than that of the first step, to form a mixture of acetates, containing mainly methyl acetate.

## Description

The present invention relates to a recovery process of methanol waste, apt to obtain a valorisation of the retrieved agents.

Methanol (CH₃OH) is the simplest of alcohols. It can be obtained in a variety of different ways, even though the most common one in the hydrogenation reaction of water gases:

CO + 2H₂ → CH₃OH

Methanol is also found among the byproducts of alcoholic fermentation, so that it is present in small amounts also in fermented drinks, such as wine. Since it is a toxic substance, removal thereof is necessary when fermented drinks are distilled (as in grappa preparation), since distillation would increase the concentration thereof to a certainly dangerous level.

Methanol is employed especially in the chemical industry. It is a good solvent, highly miscible with water; it is employed, for example, in the dilution of paints, colourings, glues, polyesters, paint removers and varnishes. It can be used as anti-freezing agent. It is also used as raw material for different uses, such as for the production of formaldehyde or of methacrylate. Another use is that as addition for fuels and another as fuel. It is also a good alkylating agent. A very peculiar use is that which is made thereof in Iceland, as a vehicle for transporting energy.

The many uses of this agent result in large amounts thereof having to be disposed of, especially of the one used as solvent.

Waste methanol can be burnt, as other solvents. Thereby, it cannot be reused, but, if heat is recovered, it can serve as fuel. In order to be disposed of in this way, it is essential that it is not mixed with too large amounts of water or with other non-flammable agents.

Another possibility is fractioned distillation and subsequent recovery. Although methanol can be separated even fully from water - unlike ethanol which forms an azeotrope - the presence therein of other agents thereby dissolved when it performs its own solvent function has as a consequence that the degree of purity normally obtained is not very high, as a so-called technical grade might be for example. This relatively low degree of purity limits and heavily affects the possibilities of use thereof as reacting agent.

One of the most important and widespread uses is in the manufacturing of acetic acid, according to the reaction:

CH₃OH + CO ---> CH₃COOH

This reaction occurs in the presence of iodidic acid and of an organo-metal compound. Firstly, a rhodium complex was used, currently an iridium complex is used.

The methanol used for this process is generally of high purity. A possible use of distilled methanol, manufactured from methanol-based waste, as seen previously, is industrially impractical, since the product which can be obtained would be of too low a purity degree for the most common uses. Moreover, purification steps would be required to obtain a product suitable for any use.

On the other hand, it is surely a disadvantage not being able to use the discarded methanol as solvent for the manufacturing reaction of acetic acid or of other agents, due to the amount of different agents present therein.

WO02/057 205 discloses a process for separating water from methanol in an initial water-methanol mixture. An azeotrope is produced, adding methylakrylate, which contains especially methanol, thus removing it from the initial mixture. The methanol-methyl akrylate azeotrope boils first and moves away from the mixture, leaving water only.

GB 1 199 120 discloses the separation of a methanol-methylacetate mixture, supplying the mixture to a first fractional column, heated by direct steam injection; the majority of the anhydrous mixture is removed as head vapour; the mixture is supplied to a second fractional column, the head whereof is again the anhydrous mixture, while the tail is pure methanol; the azeotropic mixture coming out of the head is supplied to a third fractional column, where it is added to water, having as tail a methanol-water mixture and as head a water-methylacetate mixture; finally, the methanol-water mixture of the tail is distilled and separated.

EP 2 072 489 discloses a process for the conversion of synthetic gases into ethanol and possibly into methanol.

WO96/31 456 discloses a method for improving the quality of the recycling of some residues in the manufacturing of acetic acid through methanol carbonylation, by separating alkenes and alkanes and carbonyle-containing impurities from the reaction mixture. The separation occurs by adding water and subjecting everything to a catalytic distillation.

As can be seen, none of these prior art documents provides the use of methanol contained in the waste to cause it to react with acetic acid for the final production of methylacetate.

The problem at the basis of the invention is to propose a recovery process of methanol obtained as waste following chemical processing, which overcomes the mentioned drawbacks and which allows to obtain a product of a sufficient purity for a further industrial use. This object is achieved through a recovery process of methanol waste obtained as byproducts following chemical processing which employ methanol as reacting agent and/or solvent, which provides a first step of carbonylation with CO starting from methanol, characterised in that the product of said carbonylation is caused to react in a second step with a flow of methanol waste containing methanol at a smaller concentration than that of the first step, to form an acetate mixture, containing mainly methylacetate. Methylacetate is used especially as a solvent. The dependent claims disclose preferential features of the invention.

Further features and advantages of the invention are in any case more evident from the following detailed description of a preferred embodiment, given purely as a non-limiting example and illustrated in the only attached drawing, consisting of a block diagram which illustrates the process according to the present invention, in a preferred embodiment thereof.

Referring to the same drawing, there is a reactor 1, a distillation column 2, a second reactor 3 and a second distillation column 4.

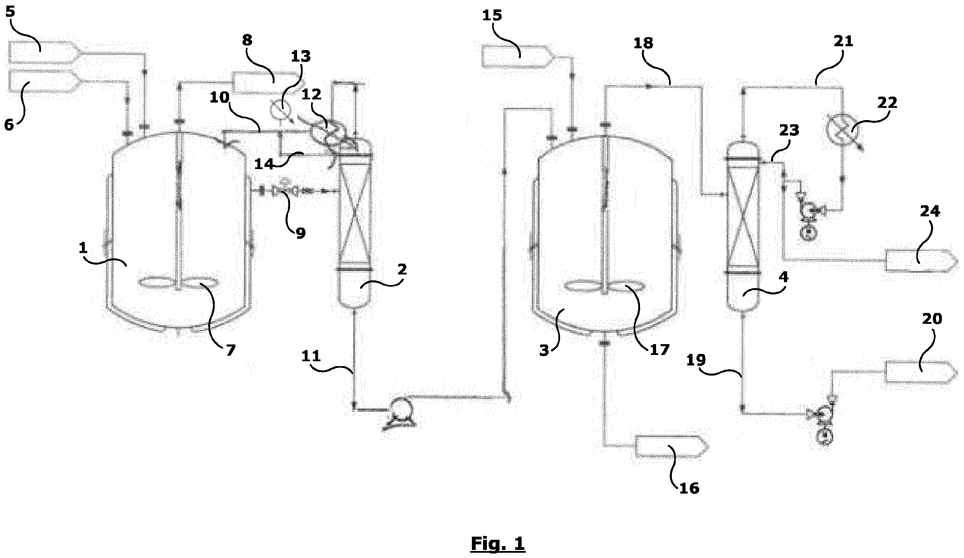

The first reactor 1 provides a CO supply 5 and a methanol supply 6. An agitator 7 is provided within reactor 1, of any known type (in the drawing a blade agitator is shown, but it could be of any other known type) . At the exit of reactor 1 a drain 8, a product-transporting pipe 9, which product is supplied to the first distillation column 2 and a recycling unit 10 are provided.

The first distillation column 2 provides, in addition to the pipe 9 which supplies it, a head leading to recycling unit 10 and a tail pipe 11. Head pipe 10 supplies a heat exchanger 12, the exit 13 of which supplies recycling unit 10 and a further recycling unit 14.

In addition to pipe 11, which supplies it, the second reactor 3 provides a methanol supply 15, a drain 16, a stirrer 17, of any known type (in the drawing a blade stirrer is shown, but it could be of any other known type) and an exit pipe 18, which supplies the second distillation column 4.

Finally, moving to the second distillation column 4, it, in addition to pipe 18 which supplies it, provides a tail pipe 19, which leads to a water drain 20, and a head pipe 21, which supplies a heat exchanger 22. Heat exchanger 22 partly supplies a recycling unit 23 and partly the finished product 24.

In the following, the process according to the present invention is described.

Carbon monoxide (CO) is supplied by supply 5 to reactor 1, while a methanol flow is supplied by supply 6. The methanol supplied by the supply 6 of the first step has a purity ranging from 95 to 99.9% by weight, preferably from 98 to 99.8% by weight. A typical composition is: methanol 99.733% by weight, methylterbutylether 0.013% by weight, tetrahydrofuran 0.020% by weight, cyclohexane 0.028% by weight, eptane 0.027% by weight, toluene 0.005% by weight, unidentified components 0.174% by weight. The methanol supplied to the first step can be pure methanol, just synthetised, or it can come from the waste solvents, after suitable purification, up to the desired level.

In reactor 1, the methanol carbonylation reaction takes place. Methanol and carbon monoxide are mixed, also by means of stirrer 7, and are maintained at a temperature of 180-200°C, under a pressure of 20-30 bar, in the presence of a catalyst. The preferred catalyst is an iridium-based catalyst, according to the Cativa process (see Jones, Platinum Metals Review, 2000, 44.3, 94-105). A rhodium-based catalyst may also be used, but conversion, selectivity and yield are lower. The catalyst is present at a concentration of 1,500-4,500 ppm, preferably at a concentration of about 3,000 ppm. Since the reaction is exothermic, a cooling system can possibly be provided in the first step, preferably of the syphon -type, with the addition of a suitable heat exchanger. The agents contained to a minor extent in supply 6 have no harmful effects on the catalyst.

Reactor 1 can operate continuously, batchwise or semi-continuously. Preferably, reactor 1 operates continuously.

Any excess gases are vented through vent 8, also in order to avoid an undesired pressure increase, while the reaction product, containing mostly acetic acid, in addition to the catalyst and components which have not reacted, comes out of reactor 1 through pipe 9 and is sent to distillation column 2, which may be a tray column, a flash distillation column or a packed bed.

Column 2 can operate continuously, batchwise or semi-continuously. Preferably, column 2 operates continuously.

In column 2 separation of acetic acid mostly from water takes place. Acetic acid has its boiling point at 118°C, while water has, as known, its boiling point at 100°C. For this reason, the head contains mostly water and is sent to heat exchanger 12 and the outgoing flow from exchanger 12 is split into two flows, one is recycled to the distillation column to further push the separation and the other, 13, is sent back to reactor 1. Acetic acid is separated in the tail and comes out of the distillation column from flow 11, which is supplied to reactor 3.

Reactor 3 provides, as already seen, a second supply 15, from which impure methanol is entered, normally to be disposed of following processes which provide it as reactant or solvent and hence impure due to the agents with which it has come into contact: the methanol supply flow 15 to the second step contains from 40 to 60% by weight of methanol, preferably around 50% by weight. A preferred composition of the methanol supply 15 to the second step is: methanol 40.01% by weight, acetone 12.39% by weight, ethanol 11.22% by weight, methylene chloride 5.46% by weight, isopropyl alcohol 4.99% by weight, acrylonitrile 4,37% by weight, ethyl acetate 4.10% by weight, tetrahydrofuran 2,49% by weight, other components 16.06% by weight. This charge generally consists of waste methanol, often as it is, sometimes roughly purified.

Some composition examples of methanol liquid wastes, used according to the present invention are the following (percentages by weight): 1) methanol 41.44%, aliphatic compounds 2.43%, acetone 11.06%, tetrahydrofuran 5.87%, ethyl acetate 2.59%, isopropyl acetate (IPAC) 0.75%, methyl ethyl ketone 0.15%, dichloromethane (MC) 3.82%, aromatic polycyclic hydrocarbons (IPA) 10.35%, ethanol 11.22%, methyl-isobutyl-ketone 2.36%, toluene 3.63%, s-buthanol (see-but) 0.10%, benzotriazole (BTA) 2.64%, n-propanol 0.07%, m-xylene 0.03%, p-xylen 0.08%, o-xylene 0.04%, n-buthanol 0.04%, dimethylformamide 0.01%, non identifiable 1.32%. 2) Methanol 48.25, aliphatic compounds 1.33, acetone 6,49%, tetrahydrofuran 2.63%, ethyl acetate 1.19%, isopropyl acetate (IPAC) 0,68%, methyl ethyl ketone 0.18%, dichloromethane (MC) 1.70%, ethanol 31.22%, methyl-isobutylketone 0.95%, toluene 2.18%, s-butanol (see-but) 0.06%, benzotriazole (BTA) 2.35%, n-propanol 0.07%, m-xilene 0.03%, p-xilene 0.08, o-xilene 0.03%, n-butanol 0.08%, non identifiables 0.5%. 3) methanol 42.58%, aliphatic compounds 2.55%, acetone 9.13%, tetrahydrofuran 5.59%, ethyl acetate 2.14%, isopropyl acetate (IPAC) 0.93%, methyl ethyl ketone 0.33%, dichloromethane (MC) 5.04%, polycyclic aromatic hydrocarbons (IPA) 9.38%, ethanol 11.87%, methyl isobutyl ketone 1.81%, toluene 4.11%, s-butanol (see-but) 0.14%, benzotriazole (BTA) 2.93%, n-propanol 0.12%, m-xilene 0.05%, p-xilene 0.17%, o-xilene 0.06%, n-butanol 0.13%, dimethylformamide 0.01%, non identifiables 0.93%. 4) methanol 48.85%, aliphatic compounds 1.93%, acetone 6.96%, tetrahydrofuran 4.07%, ethyl acetate 1.61%, isopropyl acetate (IPAC) 0.66%, methyl ethyl ketone 0.16%, dichloromethane (MC) 2.14%, aromatic polycyclic hydrocarbons (IPA) 17.48%, ethanol 7.43%, methylisobutylketone 1.42%, toluene 3.21%, s-butanol (see-but) 0.07%, benzotriazole (BTA) 2.73%, n-propanol 0.08%, m-xilene 0.03%, p-xilene 0.08%, o-xilene 0.04%, n-butanol 0.03%, dimethylformamide 0.01%, non identifiables 1.01%. 5) methanol 46.25%, aliphatic compounds 1.02%, acetone 4.40%, tetrahydrofuran 2.09%, ethyl acetate 1.05%, isopropyl acetate (IPAC) 0.45%, methyl ethyl ketone 0.12%, dichloromethane (MC) 0.84%, ethanol 38.60%, methylisobutylketone 1.10%, toluene 1.66%, s-butanol (see-but) 0.03%, benzotriazole (BTA) 1.65%, n-propanol 0.05%, m-xilene 0.02%, p-xilene 0.06%, o-xilene 0.03%, n-butanol 0.06%, non identifiables 0.52%. 6) methanol 42.54%, aliphatic compounds 2.51%, acetone 9.14%, tetrahydrofuran 5.59%, ethyl acetate 2.14%, isopropyl acetate (IPAC) 0.94%, methyl ethyl ketone 0.33%, dichloromethane (MC) 5.01% aromatic polycyclic hydrocarbons (IPA) 9.34%, ethanol 12.01%, methiisobutylketone 1.76%, toluene 4.10%, s-butanol (see-but) 0.14%, benzotriazole (BTA) 2.93%, n-propanol 0.12%, m-xilene 0.05%, p-xilene 0.17%, o-xilene 0.06%, n-butanol 0.13%, non identifiables 0.99%. 7) methanol 42.81%, aliphatic compounds 2.20%, acetone 11.46%, tetrahydrofuran 5.04%, ethyl acetate 2.10%, isopropyl acetate (IPAC) 1.01%, methyl ethyl ketone 0.36%, dichloromethane (MC) 4.21%, aromatic polycyclic hydrocarbons (IPA) 4.49%, ethanol 11.30%, methyl isobutyl ketone 1.86%, toluene 5.12%, s-butanol (see-but) 0.08%, benzotriazole (BTA) 1.95%, n-propanol 0.08%, m-xilene 0.03%, p-xilene 0.07%, o-xilene 0.02%, n-butanol 0.29%, benzotriazole glycol (BTG) 0.01%, non identifiables 5.51%. 8) methanol 44,21%, aliphatic compounds 2.01%, acetone 8.82%, tetrahydrofuran 5.12%, ethyl acetate 2.10%, isopropyl acetate (IPAC) 0.87%, methyl ethyl ketone 0.19%, dichloromethane (MC) 1.71%, aromatic polycyclic hydrocarbons (IPA) 17.56%, ethanol 9.09%, methyl isobutyl ketone 2.04%, toluene 2.62%, s-buthanol (see-but) 0.06%, benzotriazole (BTA) 2.60%, n-propanol 0.08%, m-xilene 0.04%, p-xilene 0.13%, o-xilene 0.07%, n-butanol 0.08%, non identifiables 0.6%. 9) methanol 43.28%, aliphatic compounds 1.22%, acetone 7.27%, tetrahydrofuran 2.94%, ethyl acetate 1.76%, isopropyl acetate (IPAC) 0.38%, methyl ethyl ketone 0.12%, dichloromethane (MC) 2.03%, ethanol 36.60%, methyl isobutyl ketone 0.85%, toluene 1.58%, s-butanol (sec-but) 0.38%, benzotriazole (BTA) 0.96%, n-propanol 0.08%, m-xilene 0.04%, p-xilene 0.14%, o-xilene 0.08%, n-butanol 0.02%, non identifiables 0.27%. 10) methanol 43.01%, aliphatic compounds 2.09%, acetone 9.30%, tetrahydrofuran 5.10%, ethyl acetate 1.58%, isopropyl acetate (IPAC) 1.22%, methyl ethyl ketone 0.32%, dichloromethane (MC) 3.28%, aromatic polycyclic hydrocarbons (IPA) 16.80%, ethanol 7.55%, methyl isobutyl ketone 1.94%, toluene 3.98%, s-butanol (sec-but) 0.09%, benzotriazole (BTA) 2.79%, n-propanol 0.12%, m-xilene 0.04%, p-xilene 0.10%, o-xilene 0.03%, n-butanol 0.15%, non identifiables 0.51%. 11) methanol 45.72%, aliphatic compounds 2.28%, acetone 8.06%, tetrahydrofuran 5.47%, ethyl acetate 1.52%, isopropyl acetate (IPAC) 0.68%, methyl ethyl ketone 0.28%, dichloromethane (MC) 4.28%, aromatic polycyclic hydrocarbons (IPA) 14.32%, ethanol 8.86%, methyl isobutyl ketone 1.78%, toluene 3.29%, s-butanol (sec-but) 0.06%, benzotriazole (BTA) 2.37%, n-propanol 0.06%, m-xilene 0.03%, p-xilene 0.11, o-xilene 0.01%, n-butanol 0.12%, dimethylformamide 0.01%, benzotriazole glycol (BTG) 0.01%, non identifiables 0.68%. 12) methanol 49.64%, aliphatic compounds 0.83%, acetone 6.45%, tetrahydrofuran 2.10%, ethyl acetate 1.03%, isopropyl acetate (IPAC) 0.22%, methyl ethyl ketone 0.08%, dichloromethane (MC) 1.37%, aromatic polycyclic hydrocarbons (IPA) 32.47%, methyl isobutyl ketone 0.97%, toluene 1.11%, s-butanol (sec-but) 1.27%, benzotriazole (BTA) 1.69%, n-propanol 0.02%, m-xilene 0.01%, p-xilene 0.03%, o-xilene 0.02%, n-butanol 0.11%, dimethylformamide 0.01%, benzotriazole glycol (BTG) 0.01%, non identifiables 0.56%.

Reactor 3 is made to generally operate under pressure up to 10 bar, preferably at atmospheric pressure and at a temperature ranging between 0°C and 60°C, more preferably at 35-45°C.

Reactor 3 can operate continuously, batchwise or semi-continuously. Preferably, reactor 3 operates continuously.

Any excesses are discharged through drain 16, while the mixture of esters produces comes out of reactor 3 through pipe 18. Pipe 18 supplies distillation column 4. It can be a flash column, a tray column or a packed bed. the column head supplies pipe 21 which runs through heat exchanger 22. Part of the contents of pipe 21 is sent back into column 4, through recycling pipe 23, while the finished product comes out of drain 24.

The tail of the column instead comes out through pipe 19 and is discharged in 20. The contents of discharge 20 consist mostly of excess water.

The process according to the present invention allows to retrieve waste methanol in a safe, clean manner, with a high added value, which was not possible with the processes of the prior art. The combination of the two loads containing methanol, at about 99% and 50% by weight, respectively, allows to reduce processing costs, without giving up retrieving and obtaining a product which has many different industrial uses.

Methanol waste comprising compositions from 1) to 12), seen before, have all been used for the process according to the present invention, with the same methanol removal and virtually with the same methyl acetate yield, both virtually quantitative; all this proves that the present process can be employed with methanol wastes coming from virtually any use, provided methanol concentration is about 50%.

In practice, the invention has been experimented with methanol wastes containing methanol between 41.44% and 49.64%, aliphatic compounds between 0.83% and 2.55%, acetone between 4.40% and 11.46%, tetrahydrofuran between 2.09% and 5.87%, ethyl acetate between 1.03% and 2.59%, isopropyl acetate (IPAC) between 0.22% and 1.22%, methyl ethyl ketone between 0.08% and 0.36%, dichloromethane (MC) between 0.84% and 5.04%, aromatic polycyclic hydrocarbons (IPA) between 0% and 32.47%, ethanol between 0 and 38.60%, methyl isobutyl ketone between 0.85% and 2.36%, toluene between 1.11% and 5.12%, s-butanol (sec-but) between 0.03% and 1.27%, benzotriazole (BTA) between 0.96% and 2.93%, n-propanol between 0.02% and 0.12%, m-xilene between 0.01% and 0.05%, p-xilene between 0.03% and 0.17%, o-xilene between 0.01% and 0.08%, n-butanol between 0.04% and 0.02% and 0.29%, dimethylformamide between 0% and 0.01%, benzotriazole glycol (BTG) between 0% and 0.01%, all the percentages being by weight. Also the wastes of this process, consisting mostly of water, are compatible with the most advanced ecological requirements and allow a dramatic reduction, with respect to what occurs today, of the load on the environment.

However, it is understood that the invention must not be considered limited to the particular arrangement illustrated above, which makes up only an exemplifying embodiment thereof, but that different variants are possible, all within the reach of a person skilled in the field, without departing from the scope of protection of the invention, as defined by the following claims.

### EXAMPLE

The reaction was carried out, supplying in the first step 700 kg/h of CO and 800 kg/h of methanol at 99% by weight. The reaction took place under stirring and continuously, employing an iridium-based catalyst, at a temperature of 200°C and under a pressure of 20 bar, employing a cooling system with syphon, by means of a heat exchanger.

After a distillation step, the acetic acid produced (96.77% by weight) was mixed in a second reactor with 1,450 kg/h of 50% by weight methanol, operating at 40°C and at atmospheric pressure, by stirring.
2,827 kg/h of a product were obtained, consisting 44% by weight of methyl acetate.

### LIST OF REFERENCE CHARACTERS

- 1: Reactor
- 2: Distillation column
- 3: Reactor
- 4: Distillation column
- 5: CO supply (of 1)
- 6: Methanol supply (of 1)
- 7: Stirrer (of 1)
- 8: Vent (of 1)
- 9: Pipe
- 10: Head pipe (of 2)
- 11: Tail pipe (of 2)
- 12: Heat exchanger
- 13: Output of 12
- 14: Recycling (of 2)
- 15: Methanol supply (of 3)
- 16: Vent (of 3)
- 17: Stirrer (of 3)
- 18: Pipe
- 19: Tail pipe (of 4)
- 20: Drain (of 4)
- 21: Pipe
- 22: Heat exchanger
- 23: Recycling pipe
- 24: Product discharge

## Claims

1. Process for the recovery of methanol waste obtained as a byproduct of chemical processes employing methanol as reactant and/or solvent, which provides a first carbonylation step with CO starting from methanol, **characterised in that** the product of said carbonylation is caused to react in a second step with a flow of methanol waste containing methanol at a lower concentration than that of the first step, to form a mixture of acetates, containing mainly methyl acetate.

2. Process as in 1), **characterised in that** the methanol fed by the supply (6) of the first step has a purity ranging between 95 and 99.9% by weight.

3. Process as in 2), **characterised in that** the methanol fed by the supply (6) of the first step has a purity ranging between 98 and 99.8% by weight.

4. Process as in 2) or in 3), **characterised in that** a typical composition for the first step is: methanol 99.733% by weight, methylterbutylether 0.013% by weight, tetrahydrofuran 0.020% by weight, cyclohexane 0.028% by weight, heptane 0.027% by weight, toluene 0.005% by weight, unidentified components 0.174% by weight.

5. Process as in any one of the preceding claims, **characterised in that** the methanol fed to the first step is chosen among pure, just synthesised methanol, or from waste solvents, after suitable purification, up to the desired level.

6. Process as in any one of the preceding claims, **characterised in that** the first step takes place at a temperature of 180-200°C, under a pressure of 20-30 bar, in the presence of an iridium-based or rhodium-based catalyst.

7. Process as in any one of the preceding claims, **characterised in that** the methanol supply flow (15) to the second step contains between 40 and 60% by weight of methanol.

8. Process as in 7), **characterised in that** the methanol supply flow (15) to the second step contains methanol around 50% by weight.

9. Process as in 7) or in 8), **characterised in that** the composition of the methanol supply (15) to the second step is: methanol 40.01% by weight, acetone 12.39% by weight, ethanol 11.22% by weight, methylene chloride 5.46% by weight, isopropylic alcohol 4.99% by weight, acrylonitryl 4.37% by weight, ethyl acetate 4.10% by weight, tetrahydrofuran 2.49% by weight, other components 16.06% by weight.

10. Process as in 7) or in 8), **characterised in that** the composition of the methanol supply (15) to the second step is: methanol between 41.44% and 49.64%, aliphatic compounds between 0.83% and 2.55%, acetone between 4.40% and 11.46%, tetrahydrofuran between 2.09% and 5.87%, ethyl acetate between 1.03% and 2.59, isopropyl acetate (IPAC) between 0.22% and 1.22%, methyl ethyl ketone between 0.08% and 0.36%, dichloromethane (MC) between 0.84% and 5.04%, aromatic polycyclic hydrocarbons (IPA) between 0% and 32.47%, ethanol between 0 and 38.60%, methylisobutylketone between 0.85% and 2.36%, toluene between 1.11% and 5.12%, s-butanol (sec-but) between 0.03% and 1.27%, benzotriazole (BTA) between 0.96% and 2.93%, n-propanol between 0.02% and 0.12%, m-xilene between 0.01% and 0.05%, p-xilene between 0.03% and 0.17%, o-xilene between 0.01% and 0.08%, n-butanol between 0.04% and 0.02% and 0.29%, dimethylformamide between 0% and 0.01%, benzotriazole glycol (BTG) between 0% and 0.01%, all the percentages being by weight.

11. Process as in any one of claims 7) to 10), **characterised in that** the methanol supply (15) to the second step consists of waste methanol, as such or roughly purified.

12. Process as in any one of the preceding claims, **characterised in that** the second step takes place at a pressure up to 10 bar.

13. Process as in 12), **characterised in that** the second step takes place at atmospheric pressure.

14. Process as in any one of the preceding claims, **characterised in that** the second step takes place at a temperature ranging between 0°C and 60°C.

15. Process as in 14), **characterised in that** the second step takes place at a temperature ranging between 35°C and 45°C.

16. Process as in any one of the preceding claims, **characterised in that** after each of the steps the product coming out from each of the two reactors (1; 3) undergoes a distillation step.
